(19) **Europäisches Patentamt**

**European Patent Office**

**Office européen des brevets**

(11) **EP 0 659 397 B1**

(12) **FASCICULE DE BREVET EUROPEEN**

(45) Date de publication et mention
de la délivrance du brevet:
**14.04.1999 Bulletin 1999/15**

(51) Int. Cl.$^6$: **A61K 7/13**

(21) Numéro de dépôt: **94402569.1**

(22) Date de dépôt: **14.11.1994**

(54) **Procédé de coloration directe des fibres kératiniques humaines à l'aide de colorants naturels et de vapeur d'eau**

Verfahren zur direkten Färung der menschlichen keratinischen Faser mit Hilfe natürlicher Farbstoffe und Wasserdampf

Process for direct coloration of human keratinic fibers by natural dyes and water steam

(84) Etats contractants désignés:
**AT BE CH DE ES FR GB IT LI NL SE**

(30) Priorité: **22.12.1993 FR 9315482**

(43) Date de publication de la demande:
**28.06.1995 Bulletin 1995/26**

(73) Titulaire: **L'OREAL**
**75008 Paris (FR)**

(72) Inventeurs:
• **Audousset, Marie-Pascale**
**F-92300 Levallois Perret (FR)**
• **Sturla, Jean-Michel**
**F-92210 Saint-Cloud (FR)**

(74) Mandataire:
**Andral, Christophe André Louis**
**L'OREAL**
**Centre de Recherche Charles Zviak**
**Département Propriété Industrielle**
**90, rue du Général Roguet**
**92583 Clichy Cedex (FR)**

(56) Documents cités:
**EP-A- 0 103 547      FR-A- 1 011 151**
**FR-A- 1 166 337      FR-A- 2 273 492**

• **DATABASE WPI Section Ch, Week 9003, Derwent Publications Ltd., London, GB; Class A11, AN 90-020483 & KR-A-8 901 792 (DAEWHA CO) 22 Mai 1989**
• **DATABASE WPI Section Ch, Week 8634, Derwent Publications Ltd., London, GB; Class D08, AN 86-220743 & JP-A-61 143 315 (NIPPON NATURAL KK) 1 Juillet 1986**
• **10 ed.of Condensed Chemical Dictionnary "DYE" natural**

Remarques:
Le dossier contient des informations techniques présentées postérieurement au dépôt de la demande et ne figurant pas dans le présent fascicule.

## Description

La présente invention est relative à un procédé de coloration (ou teinture) directe des fibres kératiniques mettant en oeuvre de la vapeur d'eau et une composition comprenant des colorants naturels.

La teinture des cheveux, en particulier des cheveux humains, à l'aide de colorants naturels est connue depuis l'antiquité en particulier pour le henné et le brou de noix. Ce type de teinture a été délaissé depuis de nombreuses années au profit de colorants synthétiques, en particulier les colorants d'oxydation.

Pourtant cette coloration est facile à réaliser et garantit un bon état de la fibre après teinture. Les colorants naturels sont d'autre part aisément disponibles.

On appelle colorant naturel, au sens de l'invention, tout colorant dont la structure existe telle quelle dans la nature ou dont la structure provient de l'hydrolyse ou de l'oxydation d'un produit naturel précurseur, et qui est obtenu, soit par synthèse (colorant "naturel synthétique" ), soit à partir des organismes les élaborant ou des végétaux les contenant, auquel cas il est utilisé sous forme d'extraits ou d'homogénéisats de tout ou partie de ces organismes ou végétaux.

Plusieurs raisons font que les colorants naturels sont aujourd'hui d'emploi très limité.

D'une part, ces colorants sont très sensibles au degré de sensibilisation (i.e l'état de dégradation) de la fibre : ils sont sélectifs. D'autre part, leurs puissances tinctoriales sont faibles même avec de longs temps de pause. Certains colorants naturels présentent également une faible ténacité à la lumière.

On appelle sélectivité d'un colorant la différence de montée (i.e de pouvoir de coloration) de celui-ci sur la fibre capillaire selon que celle-ci a été plus ou moins sensibilisée (i.e "abîmée") soit par un traitement tel qu'une décoloration ou une permanente, soit par les agents atmosphériques notamment dans le cas des pointes des cheveux.

D'une façon générale, les colorants naturels prennent mieux sur des cheveux légèrement sensibilisées plutôt que sur des cheveux naturels.

Les résultats de coloration obtenus sur des cheveux présentant des différences de sensibilisation sont donc hétérogènes. Ces irrégularités ne sont bien évidemment pas souhaitables d'un point de vue esthétique.

La présente invention vise à résoudre le problème ci-dessus.

La demanderesse a maintenant découvert de façon surprenante que l'utilisation d'un gaz chauffé à une température supérieure à 75°C comprenant de la vapeur d'eau sur des cheveux traités avec au moins un colorant naturel permettait d'obtenir des résultats tinctoriaux dépendant peu du degré de sensibilisation des fibres kératiniques à colorer.

Selon l'invention, les cheveux sont ainsi colorés de façon uniforme sur l'ensemble de la chevelure, des racines aux pointes, et ceci quel que soit l'état du cheveu.

La coloration est très rapide et les cheveux présentent d'excellentes propriétés cosmétiques. En outre, cette coloration présente une bonne ténacité à la lumière.

On notera que l'utilisation de la vapeur d'eau dans un procédé de coloration d'oxydation a déjà été décrite dans le brevet FR1011151, document dans lequel de la vapeur d'eau chauffée à environ 50°C est mise en oeuvre dans le but d'accélérer le processus de coloration d'oxydation des cheveux tout en diminuant les doses de teintures employées. Toutefois, à cette température, il n'y a pas de diminution de la sélectivité au sens défini ci-avant pour des colorants de type naturel.

L'utilisation de la vapeur d'eau a également été décrite dans le brevet JP61-143315, relatif à un procédé de teinture des cheveux en noir; ce procédé consiste à appliquer sur les cheveux en deux étapes successives 1°) un cataplasme à base de poudre fine de henné que l'on fait suivre d'un traitement à la vapeur d'eau destiné à fixer le henné, à la température de 50-55°C, et sous un casque entourant toute la tête pendant 45 à 90 minutes, 2°) un cataplasme à base d'un mélange de henné et d'indigo en poudre dans un rapport donné, que l'on fait suivre d'un traitement à la vapeur d'eau à 50-55°C sous un casque pendant 15 à 30 minutes pour renforcer la nuance noire.

A 55°C et dans un temps de contact inférieur à deux minutes, il n'y a pas de diminution de la sélectivité au sens défini ci-avant pour ce type de colorants.

La présente invention concerne ainsi un procédé de teinture directe des fibres kératiniques, caractérisé par le fait qu'il consiste à mettre en contact des fibres kératiniques sur lesquelles on a appliqué une composition contenant au moins un colorant direct naturel, avec un gaz contenant de la vapeur d'eau, la température dudit gaz étant supérieure à 75°C, le temps de contact entre ledit gaz et lesdites fibres à colorer étant inférieur à deux minutes.

Les colorants naturels utilisables selon l'invention sont choisis par exemple parmi les quinones hydroxylées, les indigoïdes, les hydroxyflavones, les santalines A et B, l'isatine et ses dérivés, la brasiline et son dérivé hydroxylé.

Les quinones hydroxylées sont notamment les benzoquinones, les naphtoquinones, les anthraquinones mono ou polyhydroxylées et éventuellement substituées par des groupements tels que alkyle, alkoxy, alkényle, chlore, phényle, hydroxyalkyle ou carboxy.

Les naphtoquinones sont de préférence la lawsone, la juglone, la flavioline, la naphtazarine, la naphtopurpurine, le lapachol, la plumbagine, la chloroplumbagine, la drosérone, le shikonine, la 2-hydroxy 3-méthyl 1,4-naphtoquinone, la 3,5-dihydroxy 1,4-naphtoquinone, la 2,5-dihydroxy 1,4-naphtoquinone, la 2-méthoxy 5-hydroxy 1,4-naphtoquinone, la 3-méthoxy 5-hydroxy 1,4-naphtoquinone.

Les benzoquinones sont de préférence la spinulosine, l'atromentine, l'aurentioglyocladine, la 2,5-dihydroxy 6-méthyl benzoquinone, la 2-hydroxy 3-méthyl 6-méthoxy benzoquinone, la 2,5-dihydroxy 3,6-diphényl benzoquinone, la 2,3-diméthyl 5-hydroxy 6-méthoxy benzoquinone, la 2,5-dihydroxy 6-isopropyl benzoquinone.

Les anthraquinones sont de préférence l'alizarine, la quinizarine, la purpurine, l'acide carminique, le chrysophanol, l'acide kermésique, la rhéine, l'aloeemodine, la pseudopurpurine, l'acide quinizarine carboxylique, la frangulaemodine, la 2-méthyl quinizarine, la 1-hydroxy anthraquinone, la 2-hydroxy anthraquinone.

Les indigoïdes sont de préférence l'indigo, l'indigoïne et le pourpre de Tyr.

Les hydroxyflavones sont de préférence la quercétine, la morine.

En plus de la vapeur d'eau, le gaz vecteur peut contenir de la vapeur de solvant, des gaz tels que l'oxygène, l'azote, des mélange de gaz tels que l'air ou d'autres composés vaporisables.

Les solvants utilisables pour la production de vapeur sont des solvants organiques cosmétiquement acceptables et plus particulièrement des alcools tels que l'éthanol, l'isopropanol, l'alcool benzylique, l'alcool phényléthylique ou des glycols ou éthers de glycol tels que par exemple les éthers monométhylique, monoéthylique et monobutylique de l'éthyléne glycol, le propylène glycol, le butylène glycol, le dipropylèneglycol ainsi que les alkyléthers comme le monobutyléther du diéthylèneglycol.

Le gaz comprend de préférence au moins 1% en volume de vapeur d'eau par rapport au volume total du gaz.

Le gaz est constitué de préférence soit uniquement ou essentiellement de vapeur d'eau, soit d'un mélange de vapeur d'eau et d'air.

La température du gaz est de préférence supérieure ou égale à 85°C et plus particulièrement comprise entre 85 et 150°C.

En particulier, le gaz est mis en contact avec la fibre à colorer pendant une durée allant de 0,01 seconde à 2 minutes.

De préférence, le gaz est mis en contact avec la fibre pendant une durée allant de 0,01 seconde à 30 secondes et encore plus préférentiellement de 1 à 10 secondes. L'application du gaz peut être répétée plusieurs fois sur la même fibre, chaque opération se faisant selon la durée indiquée ci-dessus.

Dans un premier mode de mise en oeuvre du procédé selon l'invention qui est ici préféré, on applique sur les cheveux une composition de coloration capillaire contenant les colorants naturels, puis on les soumet à l'action de la vapeur d'eau.

Selon d'autres modes de mise en oeuvre du procédé, il est également possible d'appliquer simultanément la composition colorante et le gaz comprenant de la vapeur d'eau.

Il est également possible de faire parvenir sur les cheveux tout ou partie de la composition colorante à l'aide du flux de gaz lorsque certains ou tous les constituants de la formule sont entraînables ou vaporisables.

Dans un mode préférée de l'invention, l'application de vapeur d'eau est suivie d'un rinçage à l'eau.

La production d'un gaz chaud comprenant de la vapeur d'eau peut se faire à l'aide de tout appareil connu en soi. Toutefois, selon la présente invention, on utilise de préférence un appareil tel que celui décrit dans la demande de brevet français FR-A-2273492, ou tout autre appareil équivalent, qui convient particulièrement bien.

La composition tinctoriale utilisée dans le procédé selon l'invention peut se présenter sous des formes habituellement utilisées pour la teinture des cheveux telles que de liquide plus ou moins épaissi ou gélifié, de crème, de mousse en aérosol ou sous toute autre forme appropriée pour réaliser une teinture des cheveux. La teneur en colorant(s) naturel(s) dans la composition colorante est généralement comprise entre 0,01 et 10% en poids par rapport au poids total de la composition.

Les compositions utilisées conformément à l'invention sont généralement des compositions aqueuses pouvant contenir des ingrédient habituellement utilisés dans les compositions cosmétiques destinées à la coloration des cheveux, tels que des solvants, des agents tensioactifs, des épaississants, des agents traitants, des agents alcalinisants ou acidifiant, des agents conservateurs, des parfums ou tout autre additif utilisé dans ce type de composition.

La composition tinctoriale contenant au moins un colorant naturel présente un pH généralement compris entre 2 et 11.

La composition peut aussi se présenter sous forme de solution anhydre ou de poudre qui sont diluées au moment de l'emploi avec de l'eau ou un support aqueux. Les poudres ainsi employées conduisent à un cataplasme. Les solutions anhydres peuvent être directement appliquées sur cheveux humides. Ces supports sont par exemple décrits dans les demandes de brevet français FR-A-2500749 et FR-A-2598318, FR-A-2526031 et FR-A-2500748.

Dans les exemples qui suivent, le support A présente la composition suivante:

| - Chlorure de béhényl triméthyl ammonium à 80% de matière active (MA) dans un mélange eau/isopropanol (15/85) | 0,5 gMA |
|---|---|
| - Farine de bois | 8 g |
| - Hydroxyéthylcellulose | 0,45 g |
| - Alcool cétylstéarylique ($C_{16}$-$C_{18}$/30-70) | 8 g |
| - Dicétylstéarate ($C_{16}$-$C_{18}$/30-70) d'éthylèneglycol | 2 g |
| - Alcool cétylstéarylique ($C_{16}$-$C_{18}$/30-70) oxyéthyléné avec 33 moles d'oxyde d'éthylène | 2 g |
| - Acide citrique        qs | pH 4 |
| - Eau        qsp | 100 g |

## EXEMPLE 1 (invention)

On utilise une composition de coloration présentant les caractéristiques suivantes :

| - Support A | 10 g |
|---|---|
| - Lawsone | 0,15 g |
| - Diméthylisosorbide vendu par la société ICI Americas sous la marque Arlasolve DMI® | 4,85 g |
| - Eau | 5 g |

On applique le mélange ci-dessus sur une mèche de cheveux naturels (mèche n°1) et sur une mèche de ces mêmes cheveux ayant subis une permanente (mèche n°2).

On envoie ensuite sur les deux mèches un jet de vapeur d'eau à 90°C pendant 45 secondes. Les mèches sont rincées puis séchées.

Les nuances sont proches. On évalue la sélectivité à partir d'une valeur relative des écarts entre les coordonnées chromatiques L, a, b, mesurées sur chacune des deux mèches avec un colorimètre MINOLTA CHROMA METER CR 200 :

$$\Delta E = \sqrt{[(L_2-L_1)^2 + (a_2-a_1)^2 + (b_2-b_1)^2]}$$

Dans cet exemple, le $\Delta E$ est de 4,8

## EXEMPLE 2 (comparatif)

On applique le mélange de l'exemple 1 sur une mèche de cheveux naturels (mèche n°1) et sur une mèche de ces mêmes cheveux ayant subis une permanente (mèche n°2).

On laisse pauser la composition pendant 30 minutes à température ambiante. Les mèches sont rincées puis séchées.

On évalue la sélectivité à partir d'une valeur relative des écarts entre les coordonnées chromatiques L, a, b, mesurées sur chacune des deux mèches avec un colorimètre MINOLTA CHROMA METER CR 200 :

$$\Delta E = \sqrt{[(L_2-L_1)^2 + (a_2-a_1)^2 + (b_2-b_1)^2]}$$

Dans cet exemple, le $\Delta E$ est de 11,8

La différence de coloration entre les 2 mèches est très importante avec ce procédé non conforme à l'invention.

Le ΔE représentant la sélectivité est beaucoup plus élevé que dans l'exemple 1 alors qu'il doit être le plus faible possible.

**EXEMPLE 3 (invention)**

On procède de la même façon qu'à l'exemple 1, à cette différence que la composition utilisée présente les caractéristiques suivantes :

| | |
|---|---|
| - Support A | 10 g |
| - 2,5-dihydroxy 1,4-naphtoquinone | 0,1 g |
| - Diméthylisosorbide vendu par la société ICI Americas sous la marque Arlasolve DMI® | 4,9 g |
| - Eau | 5 g |

On évalue la sélectivité à partir d'une valeur relative des écarts entre les coordonnées chromatiques L, a, b, mesurées sur chacune des deux mèches avec un colorimètre MINOLTA CHROMA METER CR 200 :

$$\Delta E = \sqrt{[(L_2 - L_1)^2 + (a_2 - a_1)^2 + (b_2 - b_1)^2]}$$

Dans cet exemple, le ΔE est de 2,25

**EXEMPLE 4 (comparatif)**

On procède de la même façon qu'à l'exemple 2 en utilisant la composition de l'exemple 3.

On évalue la sélectivité à partir d'une valeur relative des écarts entre les coordonnées chromatiques L, a, b, mesurées sur chacune des deux mèches avec un colorimètre MINOLTA CHROMA METER CR 200 :

$$\Delta E = \sqrt{[(L_2 - L_1)^2 + (a_2 - a_1)^2 + (b_2 - b_1)^2]}$$

Dans cet exemple, le ΔE est de 13,9

La différence de coloration entre les 2 mèches est très importante avec ce procédé non conforme à l'invention. Le ΔE représentant la sélectivité est beaucoup plus élevé que dans l'exemple 3 alors qu'il doit être le plus faible possible.

**EXEMPLE 5 (invention)**

On procède de la même façon qu'à l'exemple 1, à cette différence que la composition utilisée présente les caractéristiques suivantes :

| | |
|---|---|
| - Support A | 15 g |
| - Isatine | 0,1 g |
| - Diméthylisosorbide vendu par la société ICI Americas sous la marque Arlasolve DMI® | 4,9 g |

On évalue la sélectivité à partir d'une valeur relative des écarts entre les coordonnées chromatiques L, a, b, mesurées sur chacune des deux mèches avec un colorimètre MINOLTA CHROMA METER CR 200 :

$$\Delta E = \sqrt{[(L_2 - L_1)^2 + (a_2 - a_1)^2 + (b_2 - b_1)^2]}$$

Dans cet exemple, le $\Delta E$ est de 0,7.

**EXEMPLE 6 (comparatif)**

On procède de la même façon qu'à l'exemple 2 en utilisant la composition de l'exemple 5.

On évalue la sélectivité à partir d'une valeur relative des écarts entre les coordonnées chromatiques L, a, b, mesurées sur chacune des deux mèches avec un colorimètre MINOLTA CHROMA METER CR 200 :

$$\Delta E = \sqrt{[(L_2 - L_1)^2 + (a_2 - a_1)^2 + (b_2 - b_1)^2]}$$

Dans cet exemple, le $\Delta E$ est de 7,3.

La différence de coloration entre les 2 mèches est très importante avec ce procédé non conforme à l'invention. Le $\Delta E$ représentant la sélectivité est beaucoup plus élevé que dans l'exemple 5 alors qu'il doit être le plus faible possible.

**EXEMPLE 7 (invention)**

On procède de la même façon qu'à l'exemple 1, à cette différence que la composition utilisée présente les caractéristiques suivantes :

| | |
|---|---|
| - Support A | 15 g |
| - 2-hydroxy 3-méthyl 1,4-naphtoquinone | 0,1 g |
| - Diméthylisosorbide vendu par la société ICI Americas sous la marque Arlasolve DMI® | 4,9 g |

On évalue la sélectivité à partir d'une valeur relative des écarts entre les coordonnées chromatiques L, a, b, mesurées sur chacune des deux mèches avec un colorimètre MINOLTA CHROMA METER CR 200 :

$$\Delta E = \sqrt{[(L_2 - L_1)^2 + (a_2 - a_1)^2 + (b_2 - b_1)^2]}$$

Dans cet exemple, le $\Delta E$ est de 3,9

**EXEMPLE 8 (comparatif)**

On procède de la même façon qu'à l'exemple 2 en utilisant la composition de l'exemple 7.

On évalue la sélectivité à partir d'une valeur relative des écarts entre les coordonnées chromatiques L, a, b, mesurées sur chacune des deux mèches avec un colorimètre MINOLTA CHROMA METER CR 200 :

$$\Delta E = \sqrt{[(L_2 - L_1)^2 + (a_2 - a_1)^2 + (b_2 - b_1)^2]}$$

Dans cet exemple, le $\Delta E$ est de 10,3

La différence de coloration entre les 2 mèches est très importante avec ce procédé non conforme à l'invention. Le $\Delta E$ représentant la sélectivité est beaucoup plus élevé que dans l'exemple 7 alors qu'il doit être le plus faible possible.

**EXEMPLE 9 (invention)**

On procède de la même façon qu'à l'exemple 1, à cette différence que la composition utilisée présente les caractéristiques suivantes :

| - Support A | 15 g |
|---|---|
| - 2,5-dihydroxy 3-méthoxy 6-méthyl 1,4-benzoquinone | 0,1 g |
| - Diméthylisosorbide vendu par la société ICI Americas sous la marque Arlasolve DMI® | 4,9 g |

On évalue la sélectivité à partir d'une valeur relative des écarts entre les coordonnées chromatiques L, a, b, mesurées sur chacune des deux mèches avec un colorimètre MINOLTA CHROMA METER CR 200 :

$$\Delta E = \sqrt{[(L_2-L_1)^2 + (a_2-a_1)^2 + (b_2-b_1)^2]}$$

Dans cet exemple, le $\Delta E$ est de 1,7
Les nuances sont à peu près identiques.

**EXEMPLE 10 (comparatif)**

On procède de la même façon qu'à l'exemple 2 en utilisant la composition de l'exemple 9.
On évalue la sélectivité à partir d'une valeur relative des écarts entre les coordonnées chromatiques L, a, b, mesurées sur chacune des deux mèches avec un colorimètre MINOLTA CHROMA METER CR 200 :

$$\Delta E = \sqrt{[(L_2-L_1)^2 + (a_2-a_1)^2 + (b_2-b_1)^2]}$$

Dans cet exemple, le $\Delta E$ est de 9
La différence de coloration entre les 2 mèches est très importante avec ce procédé non conforme à l'invention. Le $\Delta E$ représentant la sélectivité est beaucoup plus élevé que dans l'exemple 9 alors qu'il doit être le plus faible possible.

## Revendications

1. Procédé cosmétique de teinture directe des fibres kératiniques, qui consiste à mettre en contact des fibres kératiniques sur lesquelles on a appliqué une composition contenant au moins un colorant naturel, c'est-à-dire tout colorant dont la structure existe telle quelle dans la nature ou dont la structure provient de l'hydrolyse ou de l'oxydation d'un produit naturel précurseur, et qui est obtenu, soit par synthèse, soit à partir des organismes les élaborant ou des végétaux les contenant, avec un gaz contenant de la vapeur d'eau, caractérisé par le fait que la température dudit gaz est supérieure à 75°C et que le temps de contact entre ledit gaz et lesdites fibres à colorer est inférieur à deux minutes.

2. Procédé selon la revendication 1, caractérisé par le fait que le gaz a une température supérieure ou égale à 85°C.

3. Procédé selon la revendication 2, caractérisé par le fait que le gaz a une température comprise entre 85 et 150°C.

4. Procédé selon l'une quelconque des revendications précédentes, caractérisé par le fait que le gaz est mis en contact avec la fibre à colorer pendant une durée allant de 0,01 seconde à 2 minutes.

5. Procédé selon la revendication 4, caractérisé par le fait que le gaz est mis en contact avec la fibre à colorer pendant une durée allant de 0,01 seconde à 30 secondes.

6. Procédé selon la revendication 5, caractérisé par le fait que le gaz est mis en contact avec la fibre à colorer pendant une durée allant de 1 seconde à 10 secondes.

7. Procédé selon l'une quelconque des revendications précédentes, caractérisé par le fait que l'application du gaz est répétée plusieurs fois sur une même fibre.

8. Procédé selon l'une quelconque des revendications précédentes, caractérisé par le fait que le gaz contient uniquement de la vapeur d'eau.

9. Procédé selon l'une quelconque des revendications 1 à 7, caractérisé par le fait que le gaz contient de la vapeur d'eau et au moins un autre composé sous forme de gaz ou de vapeur.

10. Procédé selon la revendication 9, caractérisé par le fait que le gaz contient de la vapeur d'eau et de l'air.

11. Procédé selon l'une quelconque des revendications précédentes, caractérisé par le fait que les colorants naturels sont choisis parmi les quinones hydroxylées, les indigoïdes, les hydroxyflavones, les santalines A et B, l'isatine et ses dérivés, la brasiline et son dérivé hydroxylé.

12. Procédé selon l'une quelconque des revendications précédentes, caractérisé par le fait que les colorants naturels sont présents dans des concentrations allant de 0,01 à 10% en poids par rapport au poids total de la composition.

## Claims

1. Cosmetic process for the direct dyeing of keratinous fibres, which consists in bringing keratinous fibres, to which has been applied a composition containing at least one natural dye, i.e. any dye whose structure exists per se in nature or whose structure is derived from the hydrolysis or oxidation of a natural precursor product, and which is obtained either by synthesis or from organisms which produce it or from plants containing it, into contact with a gas containing water vapour, characterized in that the temperature of the said gas is greater than 75°C and that the contact time between the said gas and the said fibres to be dyed is less than two minutes.

2. Process according to Claim 1, characterized in that the gas is at a temperature greater than or equal to 85°C.

3. Process according to Claim 2, characterized in that the gas is at a temperature between 85 and 150°C.

4. Process according to any one of the preceding claims, characterized in that the gas is brought into contact with the fibre to be dyed for a period ranging from 0.01 second to 2 minutes.

5. Process according to Claim 4, characterized in that the gas is brought into contact with the fibre to be dyed for a period ranging from 0.01 second to 30 seconds.

6. Process according to Claim 5, characterized in that the gas is brought into contact with the fibre to be dyed for a period ranging from 1 second to 10 seconds.

7. Process according to any one of the preceding claims, characterized in that application of the gas is repeated several times on the same fibre.

8. Process according to any one of the preceding claims, characterized in that the gas exclusively contains water vapour.

9. Process according to any one of Claims 1 to 7, characterized in that the gas contains water vapour and at least one other compound in gas or vapour form.

10. Process according to Claim 9, characterized in that the gas contains water vapour and air.

11. Process according to any one of the preceding claims, characterized in that the natural dyes are chosen from hydroxylated quinones, indigoids, hydroxyflavones, santalins A and B, isatin and its derivatives, and brasilin and its hydroxylated derivative.

12. Process according to any one of the preceding claims, characterized in that the natural dyes are present in concentrations ranging from 0.01 to 10% by weight relative to the total weight of the composition.

**Patentansprüche**

1. Verfahren zum direkten Färben von Keratinfasern, daß darin besteht, Keratinfasern, auf die eine Zusammensetzung aufgetragen wurde, die mindestens einen Naturfarbstoff enthält, d.h. einen beliebigen Farbstoff, dessen Struktur in der Natur vorkommt oder dessen Struktur durch Hydrolyse oder Oxidation eines natürlichen Precursors erhalten wird und der entweder synthetisch oder aus Organismen, die ihn bilden, oder aus Pflanzen, die ihn enthalten, erhalten wird, mit einem Gas, das Wasserdampf enthält, in Kontakt zu bringen, dadurch gekennzeichnet, daß die Temperatur des Gases über 75 °C liegt und die Kontaktzeit zwischen dem Gas und den zu färbenden Fasern weniger als 2 min beträgt.

2. Verfahren nach Anspruch 1, dadurch gekennzeichnet, daß das Gas eine Temperatur von mindestens 85 °C aufweist.

3. Verfahren nach Anspruch 2, dadurch gekennzeichnet, daß das Gas eine Temperatur im Bereich von 85 bis 150 °C aufweist.

4. Verfahren nach einem der vorhergehenden Ansprüche, dadurch gekennzeichnet, daß das Gas mit der zu färbenden Faser während einer Zeitdauer von 0,01 s bis 2 min in Kontakt gebracht wird.

5. Verfahren nach Anspruch 4, dadurch gekennzeichnet, daß das Gas mit der zu färbenden Faser für eine Zeitdauer von 0,01 bis 30 s in Kontakt gebracht wird.

6. Verfahren nach Anspruch 5, dadurch gekennzeichnet, daß das Gas mit der zu färbenden Faser für eine Zeitdauer von 1 bis 10 s in Kontakt gebracht wird.

7. Verfahren nach einem der vorhergehenden Ansprüche, dadurch gekennzeichnet, daß die Anwendung des Gases mehrmals an der gleichen Faser wiederholt wird.

8. Verfahren nach einem der vorhergehenden Ansprüche, dadurch gekennzeichnet, daß das Gas nur Wasserdampf enthält.

9. Verfahren nach einem der Ansprüche 1 bis 7, dadurch gekennzeichnet, daß das Gas Wasserdampf und mindestens eine weitere Verbindung in Form von Gas oder Dampf enthält.

10. Verfahren nach Anspruch 9, dadurch gekennzeichnet, daß das Gas Wasserdampf und Luft enthält.

11. Verfahren nach einem der vorhergehenden Ansprüche, dadurch gekennzeichnet, daß die Naturfarbstoffe unter den Hydroxy-chinonen, Indigoiden, Hydroxyflavonen, Santalin A und B, Isatin und seinen Derivaten und Brasilin und seinen Hydroxyderivaten ausgewählt sind.

12. Verfahren nach einem der vorhergehenden Ansprüche, dadurch gekennzeichnet, daß die Naturfarbstoffe in Konzentrationen im Bereich von 0,01 bis 10 Gew.-%, bezogen auf das Gesamtgewicht der Zusammensetzung, vorliegen.